# EUROPEAN PATENT SPECIFICATION

(11) **EP 2 997 351 B1**
(45) Date of publication and mention of the grant of the patent: **06.12.2023**
(21) Application number: 14723875.2
(22) Date of filing: 12.05.2014
(51) Int. Cl.: G01N 21/552, G01N 21/77, G01N 33/00

(54) **PLASMONIC HYDROGEN DETECTION**
PLASMONISCHE WASSERSTOFFDETEKTION
DÉTECTION PLASMONIQUE D'HYDROGÈNE

(30) Priority: 13.05.2013 GB 201308515
(43) Date of publication of application: 23.03.2016
(73) Proprietor: Kings College London, London, Greater London WC2R 2LS (GB)
(72) Inventor: ZAYATS, Anatoly, London Greater London WC2R 2LS (GB); DICKSON, Wayne, London Greater London WC2R 2LS (GB); NASIR, Mazhar Ejaz, London Greater London WC2R 2LS (PK)
(74) Representative: Script IP Limited
(86) International application number: PCT/GB2014/051448
(87) International publication number: WO 2014/184530

(56) References cited:
- WO-A1-2012/169971
- ANDREAS TITTL ET AL: "Palladium-Based Plasmonic Perfect Absorber in the Visible Wavelength Range and Its Application to Hydrogen Sensing", NANO LETTERS, vol. 11, no. 10, 30 August 2011 (2011-08-30), pages 4366-4369, XP055128990, ISSN: 1530-6984, DOI: 10.1021/nl202489g
- MAEDA E ET AL: "Array of subwavelength rectangular structures in palladium for optical hydrogen detection", PROCEEDINGS OF SPIE, vol. 8264, 9 February 2012 (2012-02-09), pages 82640N-1-82640N-5, XP055129010, ISSN: 0277-786X, DOI: 10.1117/12.907509
- NASIR M E ET AL: "Hydrogen Detected by the Naked Eye: Optical Hydrogen Gas Sensors Based on Core/Shell Plasmonic Nanorod Metamaterials", ADVANCED MATERIALS, vol. 26, no. 21, 18 March 2014 (2014-03-18), pages 3532-3537, XP055128852, ISSN: 0935-9648, DOI: 10.1002/adma.201305958
- A. V. KABASHIN ET AL: "Plasmonic nanorod metamaterials for biosensing", NATURE MATERIALS, vol. 8, no. 11, 11 October 2009 (2009-10-11), pages 867-871, XP055128856, ISSN: 1476-1122, DOI: 10.1038/nmat2546

## Description

### FIELD OF THE INVENTION

Aspects provide a plasmonic hydrogen detector and method of constructing a plasmonic hydrogen detector. There is also provided a method of detecting a change in hydrogen concentration in an environment using a plasmonic hydrogen detector in accordance with described aspects and apparatus operable to perform that method.

### BACKGROUND

Hydrogen is a widely used, but highly explosive, gas which requires only a very low concentration for combustion. Due to safety concerns and public perceptions about hydrogen, one issue of relevance is development of a reliable hydrogen detection technique. It will be appreciated that hydrogen is a gas widely used in petrochemical processing, hydrogen refuelling stations, nuclear power plants and fuel cell manufacturing. In such environments the detection of hydrogen can be an important safety concern.

Conventionally used hydrogen detection means are typically of a semiconductor type, or employ protonic conductors or simple platinum wires. Such sensors usually require electrical readings and the sensitivity of those sensors is typically enhanced at a high working temperature which can be undesirable given the explosive nature of hydrogen.

Several approaches to detecting hydrogen by using an all-optical sensor have been studied, but such approaches appear not to be able to offer a sensitive means of detection.

ANDREAS TITTL ET AL: "Palladium-Based Plasmonic Perfect Absorber in the Visible Wavelength Range and its Application to Hydrogen Sensing" NANO LETTERS, vol. II, no 10, 30 August 2011 (2011-08-30) pages 4366-4369 describes a palladium-based plasmonic perfect absorber at visible wavelengths and its application to hydrogen sensing. In particular, a simple multilayer sample design based on palladium nanowires is described. The structure exhibits near perfect absorption at visible wavelengths. The structure described utilises the plasmonic response of palladium nanowires stacked above a 200 nm thick gold film. The two layers are separated by a 65 nm dielectric MgF₂ spacer layer. When light polarized perpendicular to the wires impinges on the structure, localised plasmon oscillations are excited in the palladium wires. The resulting charge distribution leads to the antiphase oscillation of a mirror plasmon in the thick gold film below, giving rise to a circular current distribution and consequently a magnetic response. This, in turn, leads to efficient coupling of the incident light into the structure where it is dissipated as heat in the metallic components and dielectric spacer layer.

MAEDA E ET AL: "Array of subwavelength rectangular structures in palladium for optical hydrogen detection"; PROCEEDINGS OF SPIE, vol 8264, 9 February 2012 (2012-02-09) describes an array of subwavelength rectangular structures in palladium for optical hydrogen detection. A computational study of a transmittance spectrum and light wave propagation through palladium subwavelength rectangular hole arrays and rod arrays in the infrared region is described. Two structures, one a hole array, the other a rod array, are described. The structures comprise a 1 µm silicon substrate to which a 100 nm thick Pd layer was applied. The long and short sides of the holes and rectangles were fixed to 800 and 300 nm respectively. The period of the subwavelength structures was fixed to 1.1 µm. Light was incident on the silicon substrate. The transmission spectra were monitored after the light passed through the subwavelength structures. The transmittance spectrum of the hole array exhibits a strong transmission peak. The transmittance spectrum of the rectangle/rod array exhibits a transmission dip. It is described how exposure of the palladium layer to hydrogen causes a shift in the observed transmission peak and transmission dip. WO2012/169971 A1 describes a method of generating a metamaterial and the resulting metamaterial. The method described results in a metamaterial operable in the visible infrared range. The method comprises a) depositing a layer of a conductive material on a substrate; b) forming a layer of electron beam resist on the layer of conductive material; c) patterning the layer of electron beam resist using electron beam lithography to form a patterned substrate; d) depositing a layer of a noble metal on the patterned substrate; and e) removing the resist. A metamaterial operable in the visible infrared range comprising split ring resonators having a line width of about 20 nm to about 40 nm on a substrate is provided. A transparent photonic device or a sensor for chemical or biological sensing comprising the metamaterial is also described.

KABASHIN A V ET AL: "Plasmonic nanorod metamaterials for biosensing"; NATURE MATERIALS, vol. 8, 11 October 2009, pages 867-871 describes a biosensor comprising a plasmonic metamaterial consisting of an array of parallel gold nanorods oriented normally to a glass substrate to which they are attached. The gold nanorods are functionalized with receptor molecules configured to selectively bind to a target molecule of interest, such as a drug, a vitamin or a hormone.

It is desired to provide an alternative means of hydrogen detection which addresses some of the issues with existing hydrogen detectors.

### SUMMARY

Accordingly, a first aspect provides a plasmonic hydrogen detector according to claim 1.

Aspects and embodiments described recognise that use of a plasmonic metamaterial as a hydrogen detector can result in a highly sensitive detector. That sensitivity stems from the sensitivity of strong plasmonic coupling between individual nanostructure elements in the metamaterial to external perturbations, for example, as a result of a physical or chemical environmental change. Provision of a hydrogen sensitive material as an element within said metamaterial causes a change to said metamaterial in the presence of hydrogen. That change, in this instance, a change to the effective permittivity of the metamaterial can be detected by various appropriately chosen techniques.

Although it is possible to construct various hybrid hydrogen sensitive material based plasmonic systems for detection of hydrogen, for example, palladium-based plasmonic systems, such hybrid plasmonic systems are based on localised plasmon resonance sensing or surface plasmon polaritonic structures. Such systems may, for example, comprise a film or layer or a plasmonic material, onto which a film or layer of hydrogen sensitive material is placed. In some cases, the hydrogen sensitive layer, for example, palladium, may be arranged on said plasmonic material in a structured manner, for example, a grating, thus requiring sophisticated optical interrogation techniques to be used to detect hydrogen. Such structures typically require expensive lithographic fabrication techniques which makes them expensive and technologically challenging to fabricate.

Aspects and embodiments described herein recognise that the optical properties of plasmonic systems and metamaterials based on the arrangement of plasmonic components present an opportunity to design novel optical devices which may offer useful properties. Electromagnetic metamaterials can exhibit high sensitivity to their surroundings and thus can offer a means to create a sensitive detector.

Use of a plasmonic metamaterial which is sensitive to hydrogen may allow aspects and embodiments to provide a rapid, sensitive and accurate hydrogen detector. Depending upon the precise configuration of the nanostructured elements forming the metamaterial, hydrogen may be detected at low concentrations and thus a detector based upon such a metamaterial may provide a means to limit potential hazards associated with the use of the highly explosive hydrogen gas. Some aspects and embodiments may provide a hydrogen sensor which can operate to safely detect hydrogen gas at room temperature.

It will be appreciated that excitation of a plasmon may be achieved by means of appropriate radiation. Furthermore, it will be appreciated that a change to the hydrogen sensitive material can result in change in the optical properties of the metamaterial and therefore a change to reflection or transmission properties of the metamaterial may occur even off-resonance. According to the invention, the plasmonic metamaterial comprises an optical metamaterial. Accordingly, providing incident energy in the form of, for example, photons may excite surface plasmons. Whether a nanostructure is seen by interrogating incident radiation as a metamaterial is likely to depend upon the relative wavelength of the incident radiation in comparison to the spacing of adjacent nanostructure elements. In particular, it will be appreciated that for a given wavelength of interrogating incident electromagnetic radiation, the spacing of adjacent nanostructure elements may be in the region of a few tens of nanometres to several microns.

According to the invention, adjacent nanostructure elements are configured on the support such that they are electromagnetically coupled. According to the invention, the nanostructure elements are configured such that the electromagnetic field of one nanostructure element spatially overlaps that of adjacent nanostructure elements. Accordingly, rather than being seen by incident radiation as separate component elements, the nanostructure acts as a metamaterial. Arranging adjacent elements to display strong near-field interaction may ensure that the resultant structure acts as a metamaterial.

In one embodiment, the plurality of nanostructure elements are configured as an array on the support. Accordingly, that array may comprise a regular array or grid. In some embodiments, that array may comprise a quasi-regular array, such as a forest. Such a quasi-regular structure may result from structures being grown on, for example, a porous dielectric template. The pores of such a template may be located substantially regularly, as a result of employing a multi-step anodisation process. In one embodiment, the array comprises a substantially regular array.

The adjacent nanostructure elements have a spacing of the order of a few tens of nanometres to several microns, that spacing being determined by the wavelength of electromagnetic radiation selected to be incident on a resulting metamaterial. The spacing can be selected such that it is typically smaller than, but not limited to, the effective wavelength of intended interrogating radiation inside the metamaterial.

In one embodiment, the plurality of nanostructure elements are configured to provide a sub-set of nanostructure elements formed from hydrogen sensitive material interspersed amongst the plurality of nanostructure elements comprising a plasmonic material. Accordingly, entire nanostructure elements may be formed from a plasmonic material and entire nanostructure elements may be formed from a hydrogen sensitive material. The metamaterial may be configured so that those entire nanostructure elements formed from a hydrogen sensitive material are interspersed with those entire nanostructure elements formed from a plasmonic material. The relative number of hydrogen sensitive elements may be selected such that the nanostructure elements formed from a plasmonic material allow for the resulting metamaterial to exhibit plasmonic characteristics. That is to say, the concentration of hydrogen sensitive elements within the metamaterial may not be so high as to destroy overlap of electromagnetic field between adjacent plasmonic nanostructures. Nonetheless, the concentration must be selected to be sufficient to allow a resulting metamaterial to exhibit a change in permittivity in the presence of hydrogen.

In one embodiment, each of the nanostructure elements comprises: a plasmonic material and a hydrogen sensitive material. Accordingly, in some embodiments, a plurality of nanostructure elements comprise both a plasmonic material and a hydrogen sensitive material. Such an arrangement may allow for a highly hydrogen sensitive metamaterial, since an external perturbation, such as the presence of hydrogen, causes a change to the underlying metamaterial structure, and/or optical properties via a modification of the material's permittivity, as a result of the reaction of the hydrogen sensitive material forming the nanostructure elements to the presence of hydrogen.

It will be appreciated that various nanostructure elements may be used to form an appropriate metamaterial. In one embodiment, the nanostructure elements comprise a plasmonic material core. In one embodiment, the plasmonic material core comprises a hollow structure. That hollow structure may, for example, comprise a hollow tube. In one embodiment, the nanostructure elements comprise elongate elements extending from the support. In one embodiment, the nanostructure elements comprise a plasmonic material core having a hydrogen sensitive material coating. In one embodiment, the coating comprises a complete coating. In some embodiments, the coating may comprise a partial coating. In some embodiments only a proportion of said plasmonic material cores are coated in said hydrogen sensitive material. In one embodiment, the coating has a thickness a fraction of a nanometre to several hundred nanometres, that coating thickness being selected such that the thickness of the coating is less than half the spacing between individual plasmonic elements forming the metamaterial. The thickness of the coating may be chosen with desired detector sensitivity and response time in mind. Too little hydrogen sensitive material may limit the impact of hydrogen in an environment, meaning that the detector does not respond significantly to the presence of hydrogen. If the coating is, however, too thick, the response of the material in the presence of hydrogen may also be limited, the absorption of hydrogen, for example, may be limited by diffusion into the hydrogen sensitive coating.

In some embodiments, one or more of the nanostructure elements may themselves be layered. That is to say, one or more layers of hydrogen sensitive material may be interspersed with one or more layers of plasmonic material. Those layers may be of equal thickness, and/or may be configured to achieve a desired detector sensitivity.

In one embodiment, the plasmonic material comprises at least one of: copper, gold, silver or aluminium or a plasmonic doped semiconductor.

In one embodiment, the hydrogen sensitive material comprises a hydrogen absorptive material. In one embodiment, the hydrogen absorptive material comprises palladium. In one embodiment, the hydrogen absorptive material comprises a palladium nickel composite.

In one embodiment, the plasmonic metamaterial is configured to act as a bulk plasmonic waveguide. Accordingly, the resultant sensor may offer a quantitative indication of a detected concentration of hydrogen, since the sensitivity of a metamaterial acting as a waveguide may be greater than an arrangement in which only localised surface plasmons or surface plasmon polaritons are excited by incident interrogating radiation.

It will be appreciated that the plasmonic hydrogen detector according to the first aspect may be commissioned for use in a variety of detection arrangements. It may, for example, simply be located such that it is visible to the naked eye and a change in reflectivity may be detected by the human eye. That change in reflectivity, for example, an increase in reflectivity, may indicate the presence of hydrogen in an environment surrounding the plasmonic hydrogen detector. Other, more sensitive, detection arrangements may be used. In one embodiment, the plasmonic hydrogen detector further comprises a sensor operable to detect the change in permittivity of the plasmonic metamaterial in the presence of hydrogen. In one embodiment, the detector comprises one or more of: a photo diode, a ccd, a video camera, an analogue or digital camera, an optical-electrical transducer, photomultiplier or similar.. In one embodiment, the detector is operable to monitor intensity of reflected or transmitted radiation incident upon the sensor.

A second aspect provides a method of forming a plasmonic hydrogen detector according to claim 12.

Various methods to form plasmonic metamaterials may be employed. According to one embodiment, an electrochemical fabrication method may be used. Such electrochemical fabrication methods may offer a cheap and simple means to create an appropriate plurality of nanostructure elements having an appropriate configuration to be used as a plasmonic metamaterial exhibiting sensitivity to hydrogen. In one embodiment, the method of configuring the plurality of nanostructure elements on the support comprises at least one anodisation step. In one embodiment, the method of configuring the plurality of nanostructure elements on the support comprises at least one electro-deposition step. In one embodiment, the method of configuring the plurality of nanostructure elements on the support comprises at least one etching step.

According to the invention, the plasmonic metamaterial is configured to be an optical metamaterial.

According to the invention, the method comprises: configuring adjacent nanostructure elements on the support such that they are electromagnetically coupled.

According to the invention, , the method comprises: configuring nanostructure elements such that the electromagnetic field of one nanostructure element spatially overlaps that of adjacent nanostructure elements.

In one embodiment, the method comprises: configuring the plurality of nanostructure elements as an array on the support.

In one embodiment, the array comprises a substantially regular array.

In one embodiment, the adjacent nanostructure elements have a spacing of the order a few tens of nanometres to several microns, that spacing being determined by the wavelength of incident electromagnetic radiation used to interrogate the metamaterial detector. The spacing of adjacent nanostructure elements may be selected such that the spacing is typically smaller than, but not limited to, the effective wavelength of the intended interrogating radiation inside the resultant metamaterial.

In one embodiment, the method comprises: configuring the plurality of nanostructure elements to provide a sub-set of nanostructure elements formed from hydrogen sensitive material interspersed amongst the plurality of nanostructure elements comprising a plasmonic material.

In one embodiment, the method comprises: configuring each of the nanostructure elements from a plasmonic material and a hydrogen sensitive material.

In one embodiment, the nanostructure elements comprise a plasmonic material core. In one embodiment, the plasmonic material core comprises a hollow structure.

In one embodiment, the nanostructure elements comprise elongate elements extending from the support.

In one embodiment, the method comprises: coating the nanostructure elements comprising a plasmonic material core with a hydrogen sensitive material.

In one embodiment, the coating comprises a complete coating.

In one embodiment, the coating has a thickness between, for example, a fraction of a nanometre to several hundred nanometres. The coating thickness may be selected based on a desired detector sensitivity. The coating thickness may be selected such that the thickness of the coating is less than half the spacing between adjacent plasmonic nanostructure elements forming the metamaterial.

In one embodiment, the plasmonic material comprises at least one of: copper, gold, silver or aluminium or a plasmonic doped semiconductor.

In one embodiment, the hydrogen sensitive material comprises a hydrogen absorptive material.

In one embodiment, the hydrogen absorptive material comprises palladium.

In one embodiment, the plasmonic metamaterial is configured to act as a bulk plasmonic waveguide.

In one embodiment, the method further comprises providing a detector operable to detect the change in permittivity of the plasmonic metamaterial in the presence of hydrogen.

In one embodiment, the detector comprises one or more of: a photo diode, a ccd, a video camera, an analogue or digital camera, an optical-electrical transducer, photomultiplier or similar.

In one embodiment, the method comprises arranging the detector to monitor intensity of reflected or transmitted radiation incident upon the sensor.

A third aspect provides a method of detecting a change in hydrogen concentration in an environment according to claim 13.

A fourth aspect provides apparatus operable to detect a change in hydrogen concentration in an environment according to claim 14.

Further aspects, outside the scope of the claimed invention, provide: a plasmonic target-substance detector comprising:
a structure comprising a support and a plurality of nanostructure elements comprising a plasmonic material and a target-substance sensitive material; the plurality of nanostructure elements being configured on the support such that adjacent nanostructure elements are electromagnetically coupled to allow the structure to act as a plasmonic metamaterial; and wherein the target-substance sensitive material is configured to cause a change in permittivity of the plasmonic metamaterial in the presence of the target-substance.

Use of a plasmonic metamaterial as a substance detector can result in a highly sensitive detector. That sensitivity stems from the sensitivity of strong plasmonic coupling between individual nanostructure elements in the metamaterial to external perturbations, for example, as a result of a physical or chemical environmental change. Provision of a target-substance sensitive material as an element within said metamaterial causes a change to said metamateriai in the presence of the target substance. That change, in this instance, a change to the effective permittivity of the metamaterial can be detected by various appropriately chosen techniques.

According to some embodiments, the target-substance may comprise a fluid. That fluid may comprise a gas or a liquid. Such a detector may allow detection of a target substance as it flows around the nanostructure elements of the detector.

In one embodiment, the target substance may comprise a non-inert fluid. In one embodiment, the target substance may comprise a reactive fluid. In one embodiment, the target-substance sensitive material may comprise a material arranged to react with said target-substance.

In one embodiment, the target substance comprises ammonia. In one embodiment, the target-substance sensitive material comprises: an appropriately selected conducting polymer. Such a conducting polymer may, for example, comprise: polypyrrole (PPy). Polypyrrole becomes sensitive to the presence of selected gases if an appropriate functional dye is incorporates within the polymer. By way of example: for the purposes of ammonia detection, incorporation of eriochrome cyanine R (ECR) in PPy shows large changes in optical spectra via a variation of the complex refractive index when exposed to ammonia in the gas phase.

It will thus be appreciated that, outside the scope of the claimed invention, the apparatus and method of metamaterial enhanced detection described above and in the attached claims in relation to hydrogen detection may also be applied to detection of other materials, for example fluids, liquids or gases. According to such alternative arrangements, a plasmonic metamaterial is formed or "hybridised" such that a plurality of nanostructure elements are provided comprising a plasmonic material and a target-substance sensitive material. The target-substance sensitive material may comprise an appropriately selected gas-sensitive material.

### BRIEF DESCRIPTION OF THE DRAWINGS

Embodiments of the present invention will now be described further, with reference to the accompanying drawings, in which:
Figures la to 1d illustrate schematically main fabrication steps of a plasmonic metamaterial for use in some embodiments;
Figures 2a and 2b illustrate schematically a detection arrangement according to some embodiments;
Figures 3a and 3b illustrate changes to electromagnetic radiation incident on a plasmonic metamaterial for use in some embodiments;
Figures 4a and 4b show a visual change to a sensor in the presence of hydrogen according to one embodiment;
Figures 5a and 5b illustrate performance of a sensor according to one embodiment in different hydrogen concentrations; and
Figure 6 illustrates the response to laser radiation of a plasmonic metamaterial for use in some embodiments.

### DESCRIPTION OF THE EMBODIMENTS

Aspects and embodiments described herein may provide a plasmonic hydrogen detector. That plasmonic hydrogen detector is constructed from a plasmonic metamaterial. The plasmonic metamaterial itself comprises a support and a plurality of nanostructure elements. Those nanostructure elements comprise a plasmonic material and a hydrogen-sensitive material and are configured on the support to allow the structure to act as a plasmonic metamaterial. In particular, the hydrogen-sensitive material is arranged as part of the nanostructure elements forming the plasmonic material such that the presence of the hydrogen-sensitive material causes a change in permittivity of the plasmonic metamaterial resulting from the nanostructure in the presence of hydrogen.

Although it will be appreciated that an appropriate plasmonic metamaterial may be constructed in various ways, Figures la to Id illustrate schematically main fabrication steps of a plasmonic metamaterial suitable for use in some embodiments of a detector. According to one embodiment, a metamaterial can be based upon a self-assembled array of plasmonic particles each coated with a shell of hydrogen-sensitive material. One such hydrogen-sensitive material comprises palladium. Fabrication of one suitable metamaterial begins with the creation of an anodic aluminium oxide (AAO) template. That template is formed by means of anodisation of a substrate-supported aluminium thin film in, for example, sulphuric, phosphoric, or oxalic acid or any appropriate combination thereof. Such a template is shown schematically in Figure la.

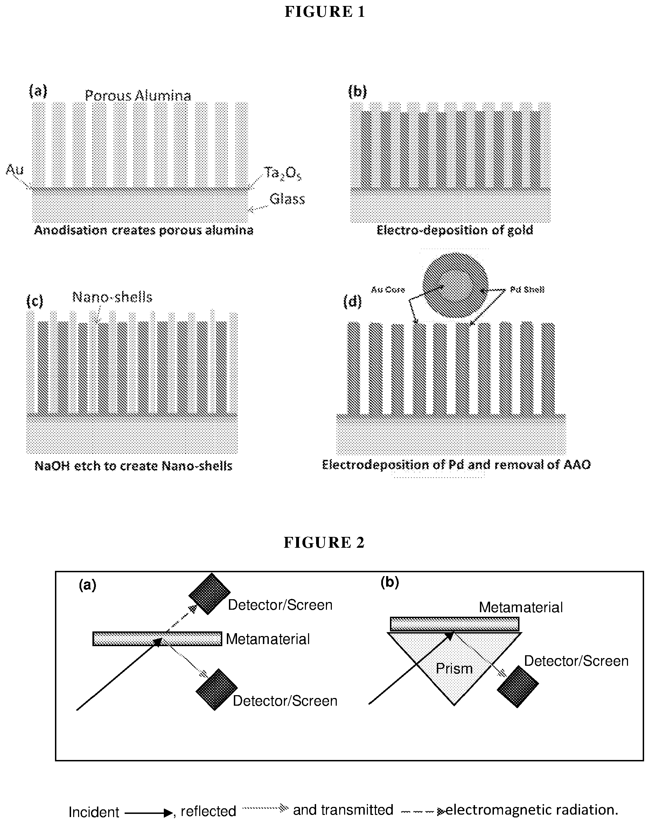

The template shown in Figure 1a comprises a glass layer on which a thin film of gold is provided and a layer of porous alumina.

After anodisation, an array of plasmonic nanoparticles is electrodeposited into pores in the anodic aluminium oxide template. The electro deposition of gold is illustrated schematically in Figure 1b. As shown in Figure lb, the pores in the anodic aluminium oxide template are substantially filled by electrodeposited gold. After the electro deposition step, a nanometric airshell is etched around each gold nanorod. That nanometric airshell is formed by exposing the anodic aluminium oxide template to dilute (30 mM) NaOH, The airshell thickness determines the final palladium shelf thickness and is determined by controlling etch time. The airshells formed around the gold nanorods are shown schematically in Figure 1c.

After forming a nanometric airshell, a hydrogen-sensitive shell material, in this example palladium, is grown around the gold nanorods by means of electrodeposition into the shell of the anodic aluminium oxide template. A final fabrication step comprises removal of the anodic aluminium oxide matrix. That removal may occur by means of etching in NaOH. Figure Id illustrates schematically a possible plasmonic metamaterial structure which can result from the fabrication method described. As shown in Figure 1d, gold nanorods are coated with a palladium shell.

It will be appreciated that, at each stage of the fabrication process described, it is possible to take steps which will have an impact upon the final form of the resulting metamaterial. For example, as described above, the etch time determines the dimension of the nanometric airshell formed around gold nanorods. Similarly, the precise dimensions of gold nanorods may be determined by electro deposition techniques employed in the second fabrication step. It will be appreciated that it is possible to create a shell surrounding a nanorod made of sacrificial material (for example, polypyrrole) to create similar plasmonic tubes. Returning then to the fabrication steps described above, such plasmonic tubes may be coated in a hydrogen- sensitive material - for example, palladium.

It will be appreciated that precise dimensions of the metamaterial formed by such a fabrication method, and in particular the dimension of the metamaterial nanostructure elements, may depend upon the particular materials employed. In the example given above, the plasmonic material chosen is gold. It will be appreciated that alternative plasmonic materials may be chosen; for example, copper, silver, aluminium or an appropriately chosen plasmonic doped semiconductor. Similarly, the hydrogen-sensitive material chosen in the example above is palladium. To a certain extent, the plasmonic material chosen for construction of a suitable plasmonic metamaterial for use in some detector embodiments is likely to determine, to a certain extent, the wavelength of operation of a hydrogen detector. That is to say, the nanostructure elements forming the plasmonic metamaterial must be configured such that they exhibit plasmon resonance at the wavelength of operation and the nanostructure elements must have a separation which is small enough that there is spatial overlap of the electric field of one nanostructure element with its adjacent, or nearest, neighbours. Provided that condition is met, incident radiation is likely to see the structure as one material rather than the component nanostructure elements. In other words, the configuration of the nanostructure elements is such that a plasmonic metamaterial is formed.

After creation of a suitable plasmonic metamaterial from appropriately configured nanostructure elements, the metamaterial is adapted such that it exhibits a change in the presence of hydrogen. In particular, a metamaterial suitable for use in some embodiments is such that a hydrogen-sensitive material is arranged within the metamaterial to cause a change in permittivity of the plasmonic metamaterial in the presence of hydrogen.

In the fabrication method described above, the nanostructure elements are coated with palladium, a hydrogen-sensitive material. The high sensitivity of a device constructed using a plasmonic material such as that described above stems from a combination of the sensitivity of each individual nanostructure element and the near field interaction between adjacent nanostructure elements. That sensitivity means that changes to the size or shape of the nanostructure elements which may be caused by stress caused as a result of a change to the hydrogen-sensitive material in relation to the presence of hydrogen causes a change, in this case, to the metamaterial complex dielectric permittivity. The physical change to the nanostructure elements may result from stress, strain, bending moment or a combination of those factors is induced as a result of a change to the hydrogen-sensitive material forming part of the metamaterial in the presence of hydrogen.

Figure 2b illustrates schematically detection arrangements according to some embodiments. In order to use a metamaterial (for example, one constructed in accordance with Figures la to 1d) as a hydrogen detector, a particular arrangement of components may be required. Two possible detection arrangements are shown in Figure 2. A hydrogen-sensitive plasmonic metamaterial constructed to perform as a hydrogen detector is provided. That metamaterial is subjected to incident radiation. Reflected radiation and/ or transmitted electromagnetic radiation may be monitored by an appropriately selected detector. If hydrogen is present in the environment surrounding metamaterial a change of properties of reflected and/ or transmitted radiation may be detected by the appropriate detectors. In some embodiments the detector may comprise the naked eye. That is to say, in some embodiments the metamaterial may exhibit a physical change in the presence of hydrogen.

A device in accordance with aspects and embodiments may be used in either configuration shown in Figure 2. In other words, the device may be used, for example, in transmission, reflection, or in attenuated total internal reflection arrangements as shown. Appropriate radiation sources and detectors for a particular wavelength or range of wavelengths of probing electromagnetic radiation may be used. In one particular arrangement developed to test the efficiency and sensitivity of an example metamaterial, the metamaterial was enclosed in a hydrogen cell.

### Empirical Testing of Example Metamaterial

Tests have been carried out using an attenuated total internal reflection detection arrangement as shown in Figure 2b. The test measurements were performed using a metamaterial in which the hydrogen-sensitive material was palladium and the plasmonic material comprised gold nanorods. Such a metamaterial was fabricated on a glass substrate and mounted onto a triangular prism using an appropriately chosen refractive index-matched solution. In this particular instance, the appropriate refractive index-matched solution comprised glycerol. The test metamaterial was illuminated at an angle above the angle of total internal reflection (θ_{inc} > θ_{c}>44 degrees), thereby facilitating excitation of bulk plasmonic waveguided modes in the nanorod layer of the metamaterial. Tests demonstrated that when hydrogen was absorbed by palladium in the metamaterial there was an accompanying expansion in lattice constant of the metamaterial. In other words, the metamaterial experienced volume expansion. When the palladium reacted chemically to form palladium hydride, the chemical reaction was accompanied by a shift of the Fermi level which resulted in a change in both the real and imaginary part of the refractive index of the metamaterial.

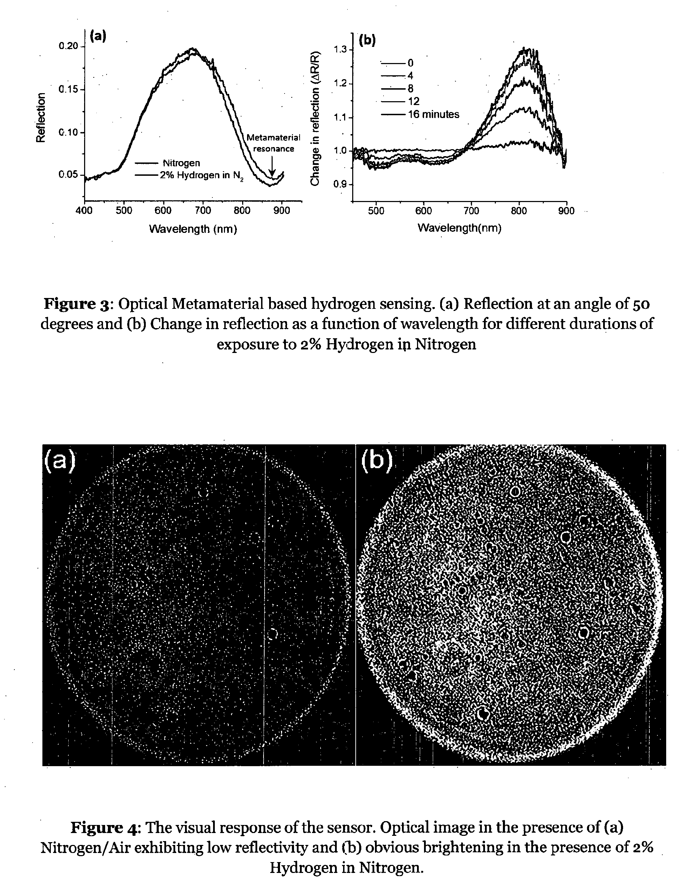

Figures 3a and 3b illustrate schematically changes to electromagnetic radiation instant on a plasmonic metamaterial for use in some embodiments. In the detection configuration shown in Figure 2b and described in relation to the tests set out above, it was determined that the reflectivity of the metamaterial sample was characterised by a strong resonant mode at a wavelength of 820 nanometres. The particular position of resonance is a function of the optical properties of each individual palladium and gold nanorod and the strong near-field interaction between them. Such an optical system exhibits a high sensitivity to changes in hydrogen concentration as a result of the physical changes which occur within the metamaterial structure on exposure to hydrogen and the corresponding complex permittivity change of, in this instance, the palladium shell of the nanostructures themselves. Both the physical change and the complex permittivity change combine to change the plasmonic properties of each individual nanostructure and modification of the palladium shell permittivity strongly modifies the optical coupling between the individual nanostructure elements forming the plasmonic metamaterial.

Figure 3b illustrates a change in intensity of reflected instant light as a function of wavelength for different durations of exposure to 2% hydrogen in a nitrogen environment surrounding a test piece of metamaterial.

The hydrogen detector properties of a metamaterial, in accordance with aspects and embodiments described, are based upon pronounced optical interactions and the optical resonances which produce extraordinary changes in the reflection from the metamaterial as a result of a shift in the resonant wavelength and a change in overall extinction.

Figures 4a and 4b show a visual change to a sensor in the presence of hydrogen according to one embodiment. It has been found that without any special effort to maximise the response of a detector in accordance with aspects and embodiments described herein, a metamaterial structure can be configured to show a 30% change in reflection at a wavelength of 820 nanometres and a spectral shift of that resonant wavelength of approximately 30 nanometres (as shown in Figure 3b). By varying the metamaterial parameters (for example, by changing nanorod dimensions, separation or thickness of the hydrogen-sensitive shell) the wavelength of operation of a metamaterial, in accordance with aspects and embodiments, can easily be tuned. As shown in Figure 4, a sensor in accordance with aspects and embodiments described herein may be capable of displaying, in a binary manner, the presence or absence of hydrogen in an environment simply by visual inspection of a metamaterial sample. On exposure to a 2% hydrogen environment, overall reflectivity of a sample metamaterial may increase dramatically, thus allowing for a quick and simple detection mechanism for hydrogen. This may be further simplified by providing a large detector metamaterial area so that no special optical interrogation methods are required.

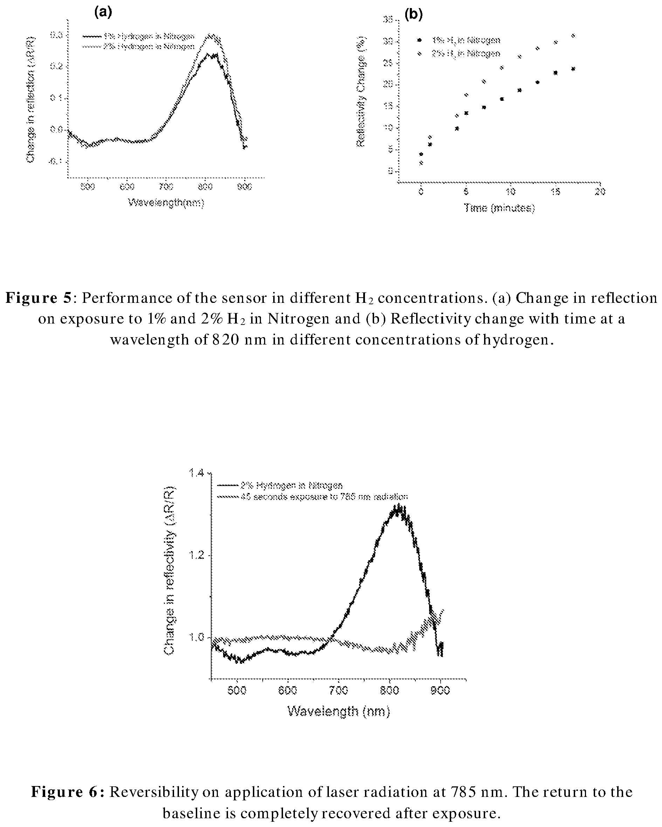

Figures 5a and 5b illustrate schematically performance of a sensor including a metamaterial such as that described in relation to Figures la to 1d. Figures 5a and 5b show the performance of such a sensor when exposed to different hydrogen concentrations. According to some embodiments, the sensitivity of a plasmonic metamaterial hydrogen detector is illustrated schematically by Figures 4a and 4b. Those Figures demonstrate the response to hydrogen in the response of a plasmonic metamaterial to hydrogen in different concentrations. At 1% concentration, the maximum change in reflectivity measured was approximately 22%. The long response time shown in Figure 5b is attributed to the larger volume of the cell encapsulating the sensor (0.2 litres) in the particular test mode used. In general, the response time is known to be a function of palladium thickness, since diffusion into palladium of any hydrogen tends to act as a dominant factor in response time. Although not demonstrated by the results from the test configuration shown in Figure 5b, nanorod arrays such as those described and shown in Figures la to 1d are expected to exhibit a much faster response than known hydrogen sensors.

Figure 6a illustrates response to laser radiation of a plasmonic metamaterial for use in a detector according to some embodiments. It has been noticed that exposure to a relatively moderate laser fluence close to the resonant wavelength of a particular metamaterial (in this case, exposure to laser radiation at 785 nanometres, when the sample under test exhibited resonance at approximately 820 nanometres) may improve the response time of a detector in accordance with aspects and embodiments. It is believed that this is a thermal effect which is expected to produce even faster performance if the exciting laser is illuminating the hydrogen-detecting metamaterial at resonance.

A metamaterial and detector in accordance with aspects and embodiments described may offer a range of possible benefits. In particular: although a metamaterial may be constructed such that it operates outside a typical optical range of incident electromagnetic radiation, the ability to use conventional optical devices including, for example, the naked eye, to detect the presence of hydrogen may offer a particularly economical hydrogen-sensing solution. Furthermore, conventional electrical detectors may present an explosive hazard since hydrogen may be ignited if it exists in an environment in a concentration of greater than 4%.

Use of a metamaterial such as that described results in a detector which is particularly sensitive to the absorption of hydrogen. That is to say, the metamaterial structure described exhibits a particular response since each palladium-coated nanorod physically and compositionally changes its individual properties as a result of interaction with hydrogen.

It is possible to change the wavelength of operation of an optical metamaterial constructed in accordance with methods described herein, since those methods offer a wider degree of fabrication freedom and therefore wavelength of operation tuneability.

Since the system can be fabricated using an electrochemical approach comprising the steps of anodisation, electro deposition and etching, the resulting hydrogen detector is relatively cheap to manufacture and readily scalable to any size.

It will be appreciated that according to some aspects and embodiments the presence of hydrogen can easily be detected by eye, by observing the brightness or colour of a sensor or detector constructed using a plasmonic metamaterial. Detection, by eye, of a change in a sensor may result in rapid hydrogen detection and may be of importance in relation to chemical plant environments where identifying the presence or otherwise of hydrogen may be particularly important.

Furthermore, providing apparatus which includes a simple integrated source and detector arrangement can readily enable quantitative detection of hydrogen. In some embodiments, such apparatus may be converted into a wireless-based sensor and enable monitoring in remote locations.

It will be appreciated that parameters of the metamaterial forming the basis of the detector device described herein may be optimized to maximise sensitivity, wavelength operation and operating speed. Parameters of the device which may be adjusted include, for example, dimensions of the nanorods, their separation, the plasmonic material chosen for construction and the hydrogen-sensitive material chosen. Furthermore, the thickness of the hydrogen-sensitive material shell may be adjusted.

Material choices may, for example, include alternative hydrogen-sensitive materials (for example, nickel palladium composites) which may improve the speed and/ or sensitivity of a palladium-based hydrogen-sensitive plasmonic metamaterial.

Further aspects and embodiments may provide a hydrogen sensor with an optical readout. It will be appreciated that some aspects and embodiments may provide a sensor which can be used as a safety device without inherent risk when exposed to high hydrogen concentrations.

It will be appreciated that hydrogen-specific sensors may be desired in a variety of commercial, domestic and academic environments. For example, fuel cell and automotive markets may require low cost hydrogen sensors. Similarly, low power sensors may be required for multiple point operations in fuel cells and in internal combustion engines. Hydrogen monitoring in power transformers is desirable. Furthermore, accurate and inexpensive hydrogen sensors immersed in oil may have considerable market potential. Further scope for deployment of aspects and embodiments described herein may exist in relation to medical diagnostic methods. Accurate hydrogen detection may be useful to determine carbohydrate breakdown deficiencies, excessive bacterial growth in the gut, lactose intolerance screening and time of passage to the small intestine.

Broadly speaking, aspects and embodiments described may provide a hydrogen detector based on an appropriate plasmonic hyperbolic metamaterial.

It will be appreciated that, outside the scope of the claimed invention, the apparatus and method of metamaterial enhanced detection described above and in the attached claims in relation to hydrogen detection may also be applied to detection of other materials, for example: other fluids, liquids or gases.

According to such alternative arrangements, a plasmonic metamaterial is formed or "hybridised" such that a plurality of nanostructure elements are provided comprising a plasmonic material and a target-substance sensitive material. The target-substance sensitive material may, for example, comprise an appropriately selected gas-sensitive material.

By way of example, detection of ammonia gas could be achieved in the following manner:
In one embodiment, outside the scope of the claimed invention, the target substance comprises ammonia and the target-substance sensitive material comprises: an appropriately selected conducting polymer. Such a conducting polymer may, for example, comprise: polypyrrole (PPy). Polypyrrole becomes sensitive to the presence of selected gases if an appropriate functional dye is incorporates within the polymer. By way of example: for the purposes of ammonia detection, incorporation of eriochrome cyanine R (ECR) in PPy shows large changes in optical spectra via a variation of the complex refractive index when exposed to ammonia in the gas phase.

Fabrication of a sensor, according to one example, may, for example, follow a chemical polymerisation route; a process that is described, for example, in Tavoli et al. (F. Tavoli and N. Alizadeh, Sensor Actuat B-Chem 176, 761 (2013)). Such a process may comprise, for example, exposing plasmonic nanostructured metamaterial elements to a solution containing both PPy and ECR to form a dye-doped polymer layer on the surface of the plasmonic nanostructure elements forming a metamaterial.

In a similar manner to the principles described in relation to hydrogen detections, the optical response of the individual plasmonic elements and the interaction between the plasmonic elements forming the metamaterial change on exposure to ammonia.

The optical scheme used to detect the presence of ammonia could then follow that described in relation to hydrogen detection.

Although illustrative embodiments of the invention have been disclosed in detail herein, with reference to the accompanying drawings, it is understood that the invention is not limited to the precise embodiment and that various changes and modifications can be effected therein by one skilled in the art without departing from the scope of the invention as defined by the appended claims.

## Claims

1. A plasmonic hydrogen detector comprising:
a structure comprising a support and a plurality of nanostructure elements comprising a plasmonic material and a separate hydrogen sensitive material that is different from the plasmonic material; said plurality of nanostructure elements being configured on said support such that adjacent nanostructure elements are electromagnetically coupled such that, on interrogation by incident radiation in the optical region, the electromagnetic field associated with one nanostructure element spatially overlaps that of adjacent nanostructure elements to allow said structure to act as a plasmonic optical metamaterial; and wherein said separate hydrogen sensitive material is configured to cause a change in permittivity of said plasmonic optical metamaterial in the presence of hydrogen.

2. A plasmonic hydrogen detector according to claim 1, wherein said plurality of nanostructure elements are configured as an array on said support.

3. A plasmonic hydrogen detector according to any preceding claim, wherein adjacent nanostructure elements have a spacing selected such that it is smaller than an effective wavelength of an intended interrogating electromagnetic radiation inside said metamaterial.

4. A plasmonic hydrogen detector according to any preceding claim, wherein said plurality of nanostructure elements are configured to provide a sub-set of nanostructure elements formed from said hydrogen sensitive material interspersed amongst a plurality of nanostructure elements comprising said plasmonic material.

5. A plasmonic hydrogen detector according to any one of claims 1 to 3, wherein each of said nanostructure elements comprises said plasmonic material and said hydrogen sensitive material.

6. A plasmonic hydrogen detector according to any preceding claim, wherein said nanostructure elements comprise one or more a plasmonic material core; plasmonic material core comprising a hollow structure; a plasmonic material core having a hydrogen sensitive material coating.

7. A plasmonic hydrogen detector according to any preceding claim, wherein said nanostructure elements comprise elongate elements extending from said support.

8. A plasmonic hydrogen detector according to any preceding claim, wherein said plasmonic material comprises at least one of: copper, gold, silver or aluminium or a plasmonic doped semiconductor.

9. A plasmonic hydrogen detector according to any preceding claim, wherein said hydrogen sensitive material comprises a hydrogen absorptive material.

10. A plasmonic hydrogen detector according to any preceding claim, further comprising a sensor operable to detect said change in permittivity of said plasmonic metamaterial in the presence of hydrogen.

11. A plasmonic hydrogen detector according to claim 10, wherein said sensor comprises one or more of: a photo diode, a ccd, a video camera, an analogue or digital camera and wherein said sensor is operable to monitor intensity of reflected or transmitted radiation incident upon said sensor.

12. A method of forming a plasmonic hydrogen detector comprising:
a structure comprising a support and a plurality of nanostructure elements comprising a plasmonic material and a separate hydrogen sensitive material that is different from the plasmonic material; said method comprising: configuring said plurality of nanostructure elements on said support such that, on interrogation by incident radiation in the optical region, adjacent nanostructure elements are electromagnetically coupled such that the electromagnetic field associated with one nanostructure element spatially overlaps that of adjacent nanostructure elements to allow said structure to act as a plasmonic optical metamaterial; and
configuring said separate hydrogen sensitive material to cause a change in permittivity of said plasmonic metamaterial in the presence of hydrogen.

13. A method of detecting a change in hydrogen concentration in an environment, said method comprising:
providing a plasmonic hydrogen detector in accordance with any one of claims 1 to 11;
arranging a source of electromagnetic radiation to be incident upon said plasmonic hydrogen detector; and
monitoring the effect of said plasmonic hydrogen detector upon said incident electromagnetic radiation.

14. Apparatus operable to detect a change in hydrogen concentration in an environment, said apparatus comprising:
a plasmonic hydrogen detector in accordance with any one of claims 1 to 11;
a source of electromagnetic radiation arranged to be incident upon said plasmonic hydrogen detector; and
an electromagnetic radiation monitor operable to monitor the effect of said plasmonic hydrogen detector upon said incident electromagnetic radiation.

## Patentansprüche

1. Plasmonischer Wasserstoff-Nachweiser, umfassend:
eine Struktur, umfassend einen Träger und eine Vielzahl von Nanostrukturelementen, umfassend ein plasmonisches Material und ein getrenntes, gegenüber Wasserstoff empfindliches Material, das sich von dem plasmonischen Material unterscheidet; wobei die Vielzahl von Nanostrukturelementen auf dem Träger konfiguriert ist, sodass benachbarte Nanostrukturelemente elektromagnetisch gekoppelt sind, sodass bei Abfrage durch einfallende Strahlung in der optischen Region, das elektromagnetische Feld, das einem Nanostrukturelement zugewiesen ist, räumlich jenes benachbarter Nanostrukturelemente überlappt, um der Struktur zu erlauben, als plasmonisches optisches Metamaterial zu wirken; und wobei das getrennte, gegenüber Wasserstoff empfindliche Material konfiguriert ist, um eine Änderung der Permittivität des plasmonischen optischen Metamaterials in Gegenwart von Wasserstoff zu bewirken.

2. Plasmonischer Wasserstoff-Nachweiser nach Anspruch 1, wobei die Vielzahl von Nanostrukturelementen als Anordnung auf dem Träger konfiguriert sind.

3. Plasmonischer Wasserstoff-Nachweiser nach einem vorstehenden Anspruch, wobei benachbarte Nanostrukturelemente eine Beabstandung aufweisen, die ausgewählt ist, sodass sie kleiner als eine effektive Wellenlänge einer bestimmten abfragenden elektromagnetischen Strahlung innerhalb des Metamaterials ist.

4. Plasmonischer Wasserstoff-Nachweiser nach einem vorstehenden Anspruch, wobei die Vielzahl von Nanostrukturelementen konfiguriert sind, um eine Teilmenge von Nanostrukturelementen bereitzustellen, die aus dem gegenüber Wasserstoff empfindlichen Material gebildet werden, das unter einer Vielzahl von Nanostrukturelementen, die das plasmonische Material umfassen durchsetzt ist.

5. Plasmonischer Wasserstoff-Nachweiser nach einem der Ansprüche 1 bis 3, wobei jedes der Nanostrukturelemente das plasmonische Material und das gegenüber Wasserstoff empfindliche Material umfasst.

6. Plasmonischer Wasserstoff-Nachweiser nach einem vorstehenden Anspruch, wobei die Nanostrukturelemente eines oder mehr umfassen aus: einem plasmonischen Materialkern; einem plasmonischen Materialkern, der eine hohle Struktur umfasst; einem plasmonischen Materialkern, der eine Beschichtung aus gegenüber Wasserstoff empfindlichem Material aufweist.

7. Plasmonischer Wasserstoff-Nachweiser nach einem vorstehenden Anspruch, wobei die Nanostrukturelemente längliche Elemente umfassen, die sich aus dem Träger erstrecken.

8. Plasmonischer Wasserstoff-Nachweiser nach einem vorstehenden Anspruch, wobei das plasmonische Material mindestens eines umfasst aus: Kupfer, Gold, Silber oder Aluminium oder einem plasmonisch dotierten Halbleiter.

9. Plasmonischer Wasserstoff-Nachweiser nach einem vorstehenden Anspruch, wobei das gegenüber Wasserstoff empfindliche Material ein Wasserstoff absorbierendes Material umfasst.

10. Plasmonischer Wasserstoff-Nachweiser nach einem vorstehenden Anspruch, weiter umfassend einen Sensor, der betriebsfähig ist, um die Änderung der Permittivität des plasmonischen Metamaterials in Gegenwart von Wasserstoff nachzuweisen.

11. Plasmonischer Wasserstoff-Nachweiser nach Anspruch 10, wobei der Sensor eines oder mehr umfasst aus: eine Photodiode, einer CCD, einer Videokamera, einer analogen oder digitalen Kamera und wobei der Sensor betriebsfähig ist, um die Intensität von reflektierter oder übertragener Strahlung, die auf den Sensor einfällt, zu überwachen.

12. Verfahren zur Bildung eines plasmonischen Wasserstoff-Nachweisers, umfassend:
eine Struktur, umfassend einen Träger und eine Vielzahl von Nanostrukturelementen, umfassend ein plasmonisches Material und ein getrenntes, gegenüber Wasserstoff empfindliches Material; wobei das Verfahren umfasst: Konfigurieren der Vielzahl von Nanostrukturelementen auf dem Träger, sodass bei Abfrage durch einfallende Strahlung in der optischen Region, benachbarte Nanostrukturelemente elektromagnetisch gekoppelt sind, sodass das elektromagnetische Feld, das einem Nanostrukturelement zugewiesen ist, räumlich jenes benachbarter Nanostrukturelemente überlappt, um der Struktur zu erlauben, als plasmonisches optisches Metamaterial zu wirken; und
Konfigurieren des getrennten, gegenüber Wasserstoff empfindlichen Materials, um eine Änderung der Permittivität des plasmonischen Metamaterials in Gegenwart von Wasserstoff zu bewirken.

13. Verfahren zum Nachweisen einer Änderung der Wasserstoffkonzentration in einer Umgebung, wobei das Verfahren umfasst:
Bereitstellen eines plasmonischen Wasserstoff-Nachweisers nach einem der Ansprüche 1 bis 11;
Anordnen einer Quelle elektromagnetischer Strahlung, die auf den plasmonischen Wasserstoff-Nachweiser einzufallen hat; und
Überwachen der Wirkung des plasmonischen Wasserstoff-Nachweisers bei einfallender elektromagnetischer Strahlung.

14. Einrichtung, die betriebsfähig ist, um eine Änderung der Wasserstoffkonzentration in einer Umgebung nachzuweisen, wobei die Einrichtung umfasst:
einen plasmonischen Wasserstoff-Nachweiser nach einem der Ansprüche 1 bis 11;
eine Quelle electromagnetischer Strahlung, die angeordnet ist, um auf auf den plasmonischen Wasserstoff-Nachweiser einzufallen; und
ein Überwachungsgerät elektromagnetischer Strahlung, das betriebsfähig ist, um die Wirkung des plasmonischen Wasserstoff-Nachweisers bei einfallender elektromagnetischer Strahlung zu überwachen.

## Revendications

1. Détecteur d'hydrogène plasmonique comprenant :
une structure comprenant un support et une pluralité d'éléments de nanostructure comprenant un matériau plasmonique et un matériau sensible à l'hydrogène séparé qui est différent du matériau plasmonique ; ladite pluralité d'éléments de nanostructure étant configurée sur ledit support de manière que des éléments de nanostructure adjacents soient couplés électromagnétiquement de manière que, lors d'une interrogation par rayonnement incident dans la région optique, le champ électromagnétique associé à un élément de nanostructure chevauche spatialement celui d'éléments de nanostructure adjacents pour permettre à ladite structure d'agir comme un métamatériau optique plasmonique ; et dans lequel ledit matériau sensible à l'hydrogène séparé est configuré pour provoquer un changement de la permittivité dudit métamatériau optique plasmonique en présence d'hydrogène.

2. Détecteur d'hydrogène plasmonique selon la revendication 1, dans lequel ladite pluralité d'éléments de nanostructure est configurée comme un réseau sur ledit support.

3. Détecteur d'hydrogène plasmonique selon une quelconque revendication précédente, dans lequel des éléments de nanostructure adjacents présentent un espacement sélectionné de manière qu'il soit inférieur à une longueur d'onde effective d'un rayonnement électromagnétique d'interrogation prévu à l'intérieur dudit métamatériau.

4. Détecteur d'hydrogène plasmonique selon une quelconque revendication précédente, dans lequel ladite pluralité d'éléments de nanostructure est configurée pour fournir un sous-ensemble d'éléments de nanostructure formés à partir dudit matériau sensible à l'hydrogène parsemés parmi une pluralité d'éléments de nanostructure comprenant ledit matériau plasmonique.

5. Détecteur d'hydrogène plasmonique selon l'une quelconque des revendications 1 à 3, dans lequel chacun desdits éléments de nanostructure comprend ledit matériau plasmonique et ledit matériau sensible à l'hydrogène.

6. Détecteur d'hydrogène plasmonique selon une quelconque revendication précédente, dans lequel lesdits éléments de nanostructure comprennent un ou plusieurs parmi : un noyau de matériau plasmonique; un noyau de matériau plasmonique comprenant une structure creuse ; un noyau de matériau plasmonique comportant un revêtement de matériau sensible à l'hydrogène.

7. Détecteur d'hydrogène plasmonique selon une quelconque revendication précédente, dans lequel lesdits éléments de nanostructure comprennent des éléments allongés s'étendant à partir dudit support.

8. Détecteur d'hydrogène plasmonique selon une quelconque revendication précédente, dans lequel ledit matériau plasmonique comprend au moins un parmi : le cuivre, l'or, l'argent ou l'aluminium ou un semi-conducteur dopé plasmonique.

9. Détecteur d'hydrogène plasmonique selon une quelconque revendication précédente, dans lequel ledit matériau sensible à l'hydrogène comprend un matériau absorbant l'hydrogène.

10. Détecteur d'hydrogène plasmonique selon une quelconque revendication précédente, comprenant en outre un capteur utilisable pour détecter ledit changement de la permittivité dudit métamatériau plasmonique en présence d'hydrogène.

11. Détecteur d'hydrogène plasmonique selon la revendication 10, dans lequel ledit capteur comprend un ou plusieurs parmi : une photodiode, un ccd, une caméra vidéo, une caméra analogique ou numérique et dans lequel ledit capteur est utilisable pour surveiller une intensité d'un rayonnement réfléchi ou transmis incident sur ledit capteur.

12. Procédé de formation d'un détecteur d'hydrogène plasmonique comprenant :
une structure comprenant un support et une pluralité d'éléments de nanostructure comprenant un matériau plasmonique et un matériau sensible à l'hydrogène séparé qui est différent du matériau plasmonique ; ledit procédé comprenant : la configuration de ladite pluralité d'éléments de nanostructure sur ledit support de manière que, lors d'une interrogation par rayonnement incident dans la région optique, des éléments de nanostructure adjacents soient couplés électromagnétiquement de manière que le champ électromagnétique associé à un élément de nanostructure chevauche spatialement celui d'éléments de nanostructure adjacents pour permettre à ladite structure d'agir comme un métamatériau optique plasmonique ; et
la configuration dudit matériau sensible à l'hydrogène séparé pour provoquer un changement de la permittivité dudit métamatériau plasmonique en présence d'hydrogène.

13. Procédé de détection d'un changement d'une concentration d'hydrogène dans un environnement, ledit procédé comprenant :
la fourniture d'un détecteur d'hydrogène plasmonique selon l'une quelconque des revendications 1 à 11 ;
l'agencement d'une source de rayonnement électromagnétique pour qu'il soit incident sur ledit détecteur d'hydrogène plasmonique ; et
la surveillance de l'effet dudit détecteur d'hydrogène plasmonique sur ledit rayonnement électromagnétique incident.

14. Appareil utilisable pour détecter un changement d'une concentration d'hydrogène dans un environnement, ledit procédé comprenant :
un détecteur d'hydrogène plasmonique selon l'une quelconque des revendications 1 à 11 ;
une source de rayonnement électromagnétique agencée pour qu'il soit incident sur ledit détecteur d'hydrogène plasmonique ; et
un dispositif de surveillance de rayonnement électromagnétique utilisable pour surveiller l'effet dudit détecteur d'hydrogène plasmonique sur ledit rayonnement électromagnétique incident.
